# EUROPÄISCHE PATENTANMELDUNG

(11) **EP 0 903 113 A2**
(43) Veröffentlichungstag der Anmeldung: **24.03.1999**
(21) Anmeldenummer: 98115861.1
(22) Anmeldetag: 22.08.1998
(51) Int. Cl.: A61B 17/80

(54) **Winkelstabile Schraubenverbindung für Knochenplatten**

(30) Priorität: 09.09.1997 AT 1516/97
(71) Anmelder: Fuchs, Werner, 2443 Loretto (AT)
(72) Erfinder: Boszotta, Harald, Dr., 2491 Steinbrunn (AT)
(74) Vertreter: Puchberger, Peter, Dipl.-Ing.

(57) **Zusammenfassung**

Bei einer winkelstabilen Schraubenverbindung für Knochenplatten am Knochen, wobei wenigstens eine Knochenschraube durch ein Schraubloch der Knochenplatte hindurchgeführt ist und eine den Kopf der Knochenschraube gegen die Knochenplatte sichernde Sicherungsschraube vorgesehen ist, wird vorgeschlagen, daß das Schraubloch (4) und der Kopf (5) der Knochenschraube (3) formschlüssig ausgebildet sind, wobei der Kopf der Knochenschraube (3) zwei zylindrische Abschnitte (7,8) aufweist, die verschiedene Durchmesser haben, und das Schraubloch (4) zwei passende zylindrische Abschnitte (9,10) aufweist.

## Beschreibung

Die Erfindung betrifft eine winkelstabile Schraubenverbindung für Knochenplatten am Knochen, wobei wenigstens eine Knochenschraube durch ein Schraublock der Knochenplatte hindurchgeführt ist und eine den Kopf der Knochenschraube gegen die Knochenplatte sichernde Sicherungsschraube vorgesehen ist.

Im Bereich der Unfallchirurgie werden Knochenplatten z.B. aus Metall als temporäre oder permanente Implantate verwendet. Die Knochenplatten werden mit Knochenschrauben am Knochen fixiert und ermöglichen so bei verschiedenen Gelenksfrakturen eine sofortige interne Fixierung der einzelnen Fragmente. Dadurch ist eine stabile Lage der einzelnen Frakturfragmente während des Wiederverheilens des Knochens gegeben. Durch die stabile Fixation der Gelenksflächen ist eine gipsfreie Nachbehandlung mit frühzeitiger Mobilisierung des Gelenkes möglich.

Bekannte derartige Knochenplatten und die zugehörigen Knochenschrauben sind in ihrer Winkelstellung zueinander nicht fixiert. Das Schraubloch der Knochenplatte weist üblicherweise eine große Senkung auf und der Kopf hat an der Kontaktfläche zumeist eine kugelflächenförmige Form. Dadurch kann die Knochenschraube innerhalb eines gewissen Winkelbereiches abweichend von der Vertikale auf die Oberfläche der Knochenplatte eingeschraubt werden.

Die DE 33 01 298 A1 offenbart nebeneinanderliegende konische Schraublöcher, in denen konisch ausgebildete Köpfe der Knochenschrauben versenkt werden. Die Köpfe werden durch eine elastische Scheibe, die von einer Schraube fixiert ist, gesichert. Diese Anordnung bietet jedoch keine tatsächlich winkelstabile Verbindung zwischen Knochenschraube und Knochenplatte, da die konischen Schraublöcher bei der geringen Dicke der Knochenplatte zu wenig Halt verleihen. Eine elastische Scheibe ist zum Fixieren der Knochenschrauben nicht geeignet.

Die US 5 364 399 A1 zeigt abgerundete Schraubenköpfe in muldenförmigen Senkungen. Die erforderliche Winkelstabilität ist dadurch nicht erzielbar, auch wenn hier die Sicherungsschraube direkt auf die Schraubenköpfe drückt. Die Druckfläche der Sicherungsschraube ist in nachteiliger Weise eben ausgebildet, wodurch es leicht zu einer Lockerung kommt und Maßtoleranzen schlecht ausgeglichen werden.

Die EP 599 640 A1 offenbart sphärisch ausgebildete Schraubenlöcher, die grundsätzlich nicht für winkelstabile Verbindungen geeignet sind und dieses Problem ist in der Druckschrift auch nicht angesprochen.

Die bekannten Konstruktionen bringen unter anderem den Nachteil mit sich, daß bei manchen Frakturformen sich Fragmente gemeinsam mit der oder den Knochenschrauben innerhalb der zugelassenen Verwinkelung bewegen können. Dies insbesondere dann, wenn sich die Knochenschrauben durch die frühe Mobilisierung lockern oder durch porotische Knochen lockern. Die Folgen sind bleibende Fehlstellungen der angrenzenden Gelenksflächen nach vollendeter Bruchheilung. Wenn in der Chirurgie, z.B. Wirbelsäulenchirurgie, Sicherungsschrauben bekannt sind, dann erfolgt durch diese keine Winkelstabilisierung der Knochenschrauben.

Die vorliegende Erfindung ist dadurch gekennzeichnet, daß das Schraubloch und der Kopf der Knochenschraube formschlüssig ausgebildet sind, wobei der Kopf der Knochenschraube zwei zylindrische Abschnitte aufweist, die verschiedene Durchmesser haben, und das Schraubloch zwei passende zylindrische Abschnitte aufweist.

Weitere vorteilhafte Merkmale der Erfindung sind den Patentansprüchen, der nachfolgenden Beschreibung und den Zeichnungen zu entnehmen.

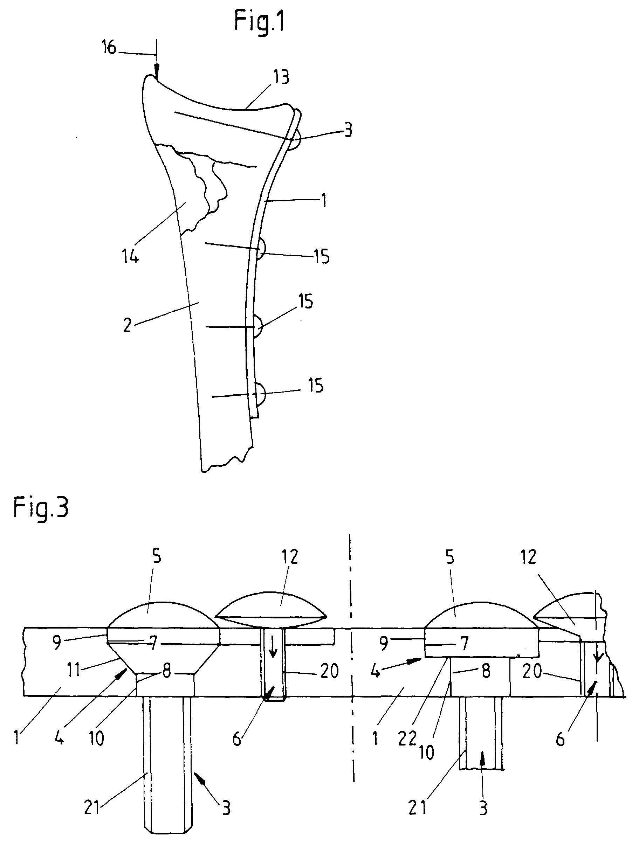

Im nachfolgenden wird die Erfindung anhand der Fig.1 bis 3 näher erläutert.

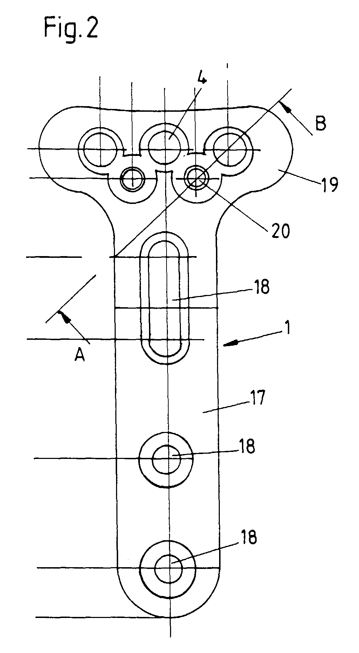

Fig.1 zeigt schematisch die Ansicht eines Knochens mit einer Fraktur und eine angeschraubte Knochenplatte. Fig.2 zeigt die Aufsicht auf eine erfindungsgemäße Knochenplatte und Fig.3 schematisch einen Schnitt nach der Linie A-B mit eingesetzten Schrauben.

In Fig.1 zeigt der Knochen 2 unterhalb der Gelenkpfanne 13 eine Frakturzone 14. Durch die mittels der Knochenschrauben 3 und 15 am Knochen fest angeschraubten Knochenplatte 1 wird der Knochen zur Verheilung stabilisiert.

Bei herkömmlichen Schraubenverbindungen für Knochenplatten kann es durch Druck in Richtung des Pfeiles 16 zu einer überstarken Belastung der Frakturzone 14 kommen, die nachgibt und wobei die Knochenschraube 3 nicht den erforderlichen Widerstand leistet, weil sich die Winkelstellung der Knochenschraube 3 zur Knochenplatte 1 verändern kann. Mit der erfindungsgemäßen Schraubenverbindung soll die erwünschte Winkelstabilität hergestellt werden.

Die Fig.2 zeigt eine Aufsicht auf die erfindungsgemäße Knochenplatte, die eine Aussenform aufweist, wie sie für derartige Knochenplatten bekannt ist. Der Schaft 17 weist Schraublöcher 18 der bekannten Art auf. Die Schraublöcher im T-Stück 19 sind hingegen besonders ausgebildet. Es sind (hier beispielsweise) drei Schraublöcher 4 für die Knochenschrauben 3 vorgesehen und versetzt zu diesen Schraublöchern sind Gewindelöcher 20 zwischen den Schraublöchern 4 vorgesehen.

Die Fig.3 zeigt den Schnitt nach der Linie A-B in Fig.2 mit eingesetzten bzw. eingeschraubten Knochen- und Sicherungsschrauben in zwei Varianten.

Der Kopf 5 der Knochenschraube 3 weist zwei zylindrische Abschnitte 7, 8 auf, die verschiedene Durchmesser haben und diese Abschnitte entsprechen zwei passenden zylindrischen Abschnitten 9,10 des Schraublochs 4. Der Übergang zwischen den beiden zylindrischen Abschnitten ist sowohl beim Kopf 5 als auch beim Schraubloch 4 durch einen kegelstumpfförmigen (konischen) Abschnitt 11 gebildet, wie in der linken Variante dargestellt. Bei der rechts gezeigten Variante ist der Übergang bei 22 stufenförmig.

Wenn die Knochenschraube 3 mit ihrem Gewinde 21 fest in den Knochen eingeschraubt wird, ergibt sich zwischen dem Kopf der Knochenschraube und der Knochenplatte 1 eine formschlüssige Verbindung, die winkelstabil ist.

Um die Knochenschraube in ihrem winkelstabilen Sitz auch dann zu fixieren, wenn sich der Sitz des Gewindes 21 im Knochen lockert, ist eine Sicherungsschraube 6 vorgesehen. Die Sicherungsschraube 6 übergreift mit ihrem Kopf 12 den Kopf 5 der Knochenschraube 3 und sichert die Knochenschraube gegen Lockerung.

Werden gemäß Fig.2 durch das T-Stück 19 drei Knochenschraube eingeschraubt, erfolgt die Sicherung durch zwei Sicherungsschrauben, die jeweils an zwei Stellen die Köpfe der Knochenschrauben übergreifen.

Die dargestellte Knochenplatte dient nur als Beispiel. Die Form und Biegung ist den jeweiligen Knochen anzupassen. Es können auch alle Schraubverbindungen gemäß Erfindung winkelstabil vorgesehen sein. Die Schraublöcher können auch in einem Winkel angeordnet sein, der verschieden von 90° ist, sodaß eine schräge Winkelstellung fixiert wird. Vorteilhafterweise sind sowohl die Knochenschrauben als auch die Sicherungsschrauben an der Oberseite ballig ausgebildet und weisen vorzugsweise den gleichen Innensechskant für Inbusschlüssel auf.

Die Unterseite der Sicherungsschrauben ist bevorzugt, entweder ballig (kugelflächig) oder konisch ausgebildet, um am Kopf der Knochenschraube eine gute Auflage zu haben.

## Patentansprüche

1. Winkelstabile Schraubenverbindung für Knochenplatten am Knochen, wobei wenigstens eine Knochenschraube durch ein Schraubloch der Knochenplatte hindurchgeführt ist und eine den Kopf der Knochenschraube gegen die Knochenplatte sichernde Sicherungsschraube vorgesehen ist, dadurch gekennzeichnet, daß das Schraubloch (4) und der Kopf (5) der Knochenschraube (3) formschlüssig ausgebildet sind, wobei der Kopf der Knochenschraube (3) zwei zylindrische Abschnitte (7,8) aufweist, die verschiedene Durchmesser haben, und das Schraubloch (4) zwei passende zylindrische Abschnitte (9,10) aufweist.

2. Schraubenverbindung nach Anspruch 1, dadurch gekennzeichnet, daß der Übergang zwischen den beiden zylindrischen Abschnitten (7,8,9,10) am Kopf und im Schraubloch durch einen kegelstumpfförmigen Abschnitt (11) gebildet ist.

3. Schraubenverbindung nach Anspruch 1, dadurch gekennzeichnet, daß der Übergang zwischen den beiden zylindrischen Abschnitten (7, 8, 9, 10) am Kopf und im Schraubloch stufenförmig (22) ausgebildet ist.

4. Schraubenverbindung nach einen der Ansprüche 1 bis 3, dadurch gekennzeichnet, daß die Sicherungsschraube (6) mit ihrem Kopf (12) in an sich bekannter Weise den Kopf (5) der Knochenschraube (3) abschnittweise übergreift und daß die Unterseite der Sicherungsschraube (6) ballig oder konisch ausgebildet ist.
